# EUROPÄISCHE PATENTANMELDUNG

(11) **EP 4 042 938 A1**
(43) Veröffentlichungstag der Anmeldung: **17.08.2022**
(21) Anmeldenummer: 21157162.5
(22) Anmeldetag: 15.02.2021
(51) Int. Cl.: A61B 5/055

(54) **MAGNETRESONANZVORRICHTUNG MIT EINER REINIGUNGSEINHEIT, SOWIE EINEM VERFAHREN ZU EINEM REINIGEN EINES PATIENTENAUFNAHMEBEREICHS**

(71) Anmelder: Siemens Healthcare GmbH, 91052 Erlangen (DE)
(72) Erfinder: Gügel-Wild, Stefanie, 91094 Langensendelbach (DE); Köferl, Marianne, 95686 Fichtelberg (DE); Maciejewski, Bernd, 91443 Scheinfeld (DE); Schneider, Michael, 91056 Erlangen (DE); Stein, Annette, 91080 Spardorf (DE)

(57) **Zusammenfassung**

Die Erfindung geht aus von einer Magnetresonanzvorrichtung mit einer Scannereinheit, einem von der Scannereinheit zumindest teilweise umgebenen Patientenaufnahmebereich, wobei die Scannereinheit eine den Patientenaufnahmebereich zumindest teilweise umgebende Umhausung umfasst, und einer Patientenlagerungsvorrichtung mit einem Patiententisch, wobei der Patiententisch zu einer horizontalen Bewegung innerhalb des Patientenaufnahmebereichs ausgebildet ist, wobei die Magnetresonanzvorrichtung eine Reinigungseinheit zur Reinigung der den Patientenaufnahmebereich umgebenden Umhausung aufweist, wobei die Reinigungseinheit zumindest teilweise am Patiententisch angeordnet ist.

## Beschreibung

Die vorliegende Erfindung betrifft eine Magnetresonanzvorrichtung mit einer Scannereinheit, einem von der Scannereinheit zumindest teilweise umgebenen Patientenaufnahmebereich, wobei die Scannereinheit eine den Patientenaufnahmebereich zumindest teilweise umgebende Umhausung umfasst, und einer Patientenlagerungsvorrichtung mit einem Patiententisch, wobei der Patiententisch zu einer horizontalen Bewegung innerhalb des Patiententischs ausgebildet ist. Des Weiteren betrifft die vorliegende Erfindung auch ein Verfahren zu einem Reinigen eines Patientenaufnahmebereichs, insbesondere einer den Patientenaufnahmebereich umgebenden Umhausung, einer Magnetresonanzvorrichtung mittels einer Reinigungseinheit.

Während Magnetresonanzuntersuchungen, bei denen der Patient mit dem Kopf voran in den Patientenaufnahmebereich eingebracht und/oder eingefahren wird, befindet sich der Kopf des Patienten während der gesamten Dauer der Magnetresonanzuntersuchung innerhalb des Patientenaufnahmebereichs. Aufgrund einer baulichen Ausbildung des Patientenaufnahmebereichs mit einem Durchmesser von ca. 60 cm bis 80 cm und einer Länge von 1,2 m bis 1,8 m befindet sich dabei der Kopf des Patienten relativ nahe zu einer den Patientenaufnahmebereich umgebenden Umhausung der Magnetresonanzvorrichtung. Hierdurch kann allein schon durch ein Atmen und/oder ein Husten usw. des Patienten eine Verunreinigung und/oder eine Kontamination des Patientenaufnahmebereichs und/oder auch des Patiententischs erfolgen.

Eine Reinigung des Patiententischs und der den Patientenaufnahmebereichs umgebenden Umhausung gestaltet sich sehr unterschiedlich. Während der Patiententisch in einem bezüglich des Patientenaufnahmebereichs herausgefahrenen Zustand für ein Reinigungspersonal frei zugänglich ist und somit einfach gereinigt werden kann gestaltet sich die Reinigung des Patientenaufnahmebereichs, insbesondere die den Patientenaufnahmebereich umgebende Umhausung, aufgrund der Länge des Patientenaufnahmebereichs besonders schwierig. Bei einer Länge der Magnetresonanzvorrichtung und damit auch des Patientenaufnahmebereichs von 1,5 m und mehr ist es sehr schwierig für das Reinigungspersonal, die verunreinigten Stellen und/oder die kontaminierten Stellen innerhalb des Patientenaufnahmebereichs, insbesondere der den Patientenaufnahmebereich umgebenden Umhausung, zu erreichen.

Der vorliegenden Erfindung liegt insbesondere die Aufgabe zugrunde, eine einfache und zeitsparende Reinigung des Patientenaufnahmebereichs einer Magnetresonanzvorrichtung zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzvorrichtung mit einer Scannereinheit, einem von der Scannereinheit zumindest teilweise umgebenen Patientenaufnahmebereich, wobei die Scannereinheit eine den Patientenaufnahmebereich zumindest teilweise umgebenden Umhausung umfasst, und einer Patientenlagerungsvorrichtung mit einem Patiententisch, wobei der Patiententisch zu einer horizontalen Bewegung innerhalb des Patientenaufnahmebereichs ausgebildet ist. Erfindungsgemäß weist die Magnetresonanzvorrichtung eine Reinigungseinheit zur Reinigung der den Patientenaufnahmebereich umgebenden Umhausung auf, wobei die Reinigungseinheit zumindest teilweise am Patiententisch angeordnet ist.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu die Scannereinheit. Die Scannereinheit der Magnetresonanzvorrichtung umfasst bevorzugt eine Detektoreinheit, insbesondere eine Magneteinheit, zur Erfassung der medizinischen und/oder diagnostischen Bilddaten. Bevorzugt umfasst hierbei die Scannereinheit, insbesondere die Magneteinheit, einen Grundmagneten, ein Gradienten-System und eine Hochfrequenzantenneneinheit. Die Hochfrequenzantenneneinheit ist fest innerhalb der Scannereinheit angeordnet und zur Aussendung eine Anregungspulses ausgelegt und/oder ausgebildet. Zur Erfassung der Magnetresonanzsignale weist die Magnetresonanzvorrichtung lokale Hochfrequenzantenneneinheiten auf, die um den zu untersuchenden Bereich des Patienten angeordnet werden.

Der Grundmagnet ist zur Erzeugung eines homogenen Grundmagnetfelds mit einer definierten und/oder bestimmten Magnetfeldstärke, wie beispielsweise mit einer definierten und/oder bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Das Gradienten-System ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Beispielsweise ist hierzu der Patientenaufnahmebereich zylinderförmig ausgebildet und/oder zylinderförmig von der Scannereinheit umgeben. Hierzu weist die Scannereinheit eine den Patientenaufnahmebereich zumindest teilweise umgebenden Umhausung auf. Bevorzugt umgibt die Umhausung der Scannereinheit den Patientenaufnahmebereich zylinderförmig. Die Umhausung kann hierbei auch eine dem Patientenaufnahmebereich zugewandte Seite der Hochfrequenzantenneneinheit umfassen und/oder einstückig mit der dem Patientenaufnahmebereich zugewandte Seite der Hochfrequenzantenneneinheit ausgebildet sein.

Innerhalb des Patientenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereichs weist die Magnetresonanzvorrichtung die Patientenlagerungsvorrichtung auf. Die Patientenlagerungsvorrichtung weist bevorzugt den bewegbaren Patiententisch auf. Der Patiententisch ist zu einer Lagerung des Patienten ausgebildet. Für eine Magnetresonanzuntersuchung wird der Patient zunächst auf dem Patiententisch der Patientenlagerungsvorrichtung positioniert und anschließend der Patiententisch zusammen mit dem Patienten derart innerhalb des Patientenaufnahmebereichs positioniert, bis der zu untersuchende Bereich des Patienten innerhalb des FOVs und/oder des Isozentrums der Magnetresonanzvorrichtung, insbesondere der Scannereinheit, angeordnet ist. Der Patiententisch ist hierbei in horizontaler Richtung, insbesondere in Einfahrrichtung, in den Patientenaufnahmebereich bewegbar ausgebildet und/oder gelagert.

Die Reinigungseinheit ist bevorzugt zu einer Reinigung des Patientenaufnahmebereichs, insbesondere der den Patientenaufnahmebereich umgebenden Umhausung, ausgebildet. Die Reinigungseinheit ist zumindest teilweise am Patiententisch angeordnet, so dass während einer horizontalen Bewegung des Patiententischs ein Reinigungsvorgang gestartet und/oder durchgeführt werden kann. Bevorzugt wird hierbei nach einer Magnetresonanzuntersuchung an einem Patienten der Patiententisch zur Reinigung des Patientenaufnahmebereichs, insbesondere der den Patientenaufnahmebereich umgebenden Umhausung, in den Patientenaufnahmebereich eingefahren. Bevorzugt weist die Reinigungseinheit mehrere Module und/oder Einheiten auf, wobei zumindest eine dieser Einheiten und/oder Module am Patiententisch angeordnet sind.

Die Reinigungseinheit kann dabei derart ausgebildet sein, dass der Reinigungsvorgang zumindest teilweise automatisch und/oder selbsttätig durch die Reinigungseinheit, insbesondere nach einem Beenden einer Magnetresonanzuntersuchung, erfolgen kann. Bevorzugt weist hierbei die Reinigungseinheit eine Steuerungseinheit und/oder eine Recheneinheit auf, die zu einer Steuerung einer Ausführung des Reinigungsvorgangs mittels der Reinigungseinheit ausgebildet ist.

Die Erfindung ermöglicht eine einfache und zeitsparende Reinigung der den Patientenaufnahmebereich umgebenden Umhausung der Magnetresonanzvorrichtung. Insbesondere kann aufgrund der zumindest teilweisen Anordnung der Reinigungseinheit am Patiententisch eine gute Erreichbarkeit von verunreinigten und/oder kontaminierten Bereichen innerhalb des Patientenaufnahmebereichs ermöglicht werden und damit eine effiziente Reinigung der den Patientenaufnahmebereich umgebenden Umhausung erreicht werden. Bevorzugt wird hierbei ein Reinigungsvorgang mittels der Reinigungseinheit während einer horizontalen Bewegung des Patiententischs, insbesondere einem Einfahren des Patiententischs in den Patientenaufnahmebereich und/oder einem Ausfahren des Patiententischs aus dem Patientenaufnahmebereichs, durchgeführt.

In einer vorteilhaften Weiterbildung der erfindungsgemäßen Magnetresonanzvorrichtung kann es vorgesehen sein, dass die Reinigungseinheit zumindest eine Einheit aufweist, die innerhalb des Patiententischs angeordnet ist und/oder als Zusatzeinheit auf dem Patiententisch anordbar und/oder positionierbar ist. Unter einer Einheit, die innerhalb des Patiententischs angeordnet ist, soll hierbei insbesondere eine Einheit der Reinigungseinheit verstanden werden, die in einem von einem Gehäuse des Patiententischs umschlossenen Bereich angeordnet ist. Unter einer auf dem Patiententisch anordbaren und/oder positionierbaren Zusatzeinheit soll hierbei insbesondere verstanden werden, dass die Zusatzeinheit nur für Reinigungszwecke auf dem Patiententisch angeordnet ist und/oder positioniert ist und während einer Magnetresonanzuntersuchung an einem Patienten von dem Patiententisch demontiert und/oder abgebaut ist. Derart kann eine besonders platzsparende Anordnung der Reinigungseinheit bereitgestellt werden. Zudem kann derart auch eine Beeinträchtigung einer Lagerungsfläche des Patiententischs zur Lagerung des Patienten vorteilhaft verhindert werden. Insbesondere kann durch eine derartige Anordnung der Reinigungseinheit am Patiententisch eine Beschränkung eines zur Verfügung stehenden Platzes und/oder Raums innerhalb des Patientenaufnahmebereichs für einen Patienten während einer Magnetresonanzuntersuchung vorteilhaft verhindert werden. Zudem können durch die Ausbildung der Reinigungseinheit als Zusatzeinheit, die für eine Reinigung des den Patientenaufnahmebereich umgebenden Umhausung auf dem Patiententischs anordbar und/oder positionierbar ist, auch bereits bestehende und/oder in Gebrauch befindliche Systeme von Magnetresonanzvorrichtungen und/oder von Patientenlagerungsvorrichtungen mit einer Reinigungseinheit besonders einfach nachgerüstet werden. Derart kann auch eine Reinigung der den Patientenaufnahmebereich umgebenden Umhausung konstruktiv einfach erreicht und/oder bereitgestellt werden.

In einer vorteilhaften Weiterbildung der erfindungsgemäßen Magnetresonanzvorrichtung kann es vorgesehen sein, dass die Reinigungseinheit eine Sprüheinheit aufweist, wobei die Sprüheinheit Düsen aufweist zu einem Verteilen eines Reinigungsmittels. Das Reinigungsmittel umfasst bevorzugt eine Reinigungsflüssigkeit, so dass das Verteilen des Reinigungsmittels ein Versprühen der Reinigungsflüssigkeit umfasst. Vorzugsweise ist die Sprüheinheit, insbesondere die Düsen der Sprüheinheit derart am Patiententisch angeordnet, dass bei der Verteilung des Reinigungsmittels das Reinigungsmittel auf die Umhausung des Patientenaufnahmebereichs auftrifft. Insbesondere sind hierbei die Düsen der Sprüheinheit in dem Patiententisch integriert und/oder auf diesem als Zusatzeinheit angeordnet. Die Düsen können dabei nach oben, insbesondere auf eine einer Lagerungsfläche des Patiententischs zugwandten Seite der Umhausung des Patientenaufnahmebereichs, gerichtet sein, so dass bevorzugt ein Bereich der Umhausung des Patientenaufnahmebereichs, der während einer Magnetresonanzuntersuchung einer hohen Wahrscheinlichkeit einer Kontaminierung und/oder Verunreinigung, beispielsweise durch Atmen und/oder Husten des Patienten, ausgesetzt ist, bei der Verteilung des Reinigungsmittels, insbesondere dem Versprühen der Reinigungsflüssigkeit, erfasst wird. Diese Ausgestaltung der Erfindung ermöglicht eine einfache und effiziente, insbesondere flächendeckende, Verteilung eines Reinigungsmittels, insbesondere einer Reinigungsflüssigkeit, innerhalb des Patientenaufnahmebereichs, insbesondere an eine den Patientenaufnahmebereich umgebende Umhausung.

In einer vorteilhaften Weiterbildung der erfindungsgemäßen Magnetresonanzvorrichtung kann es vorgesehen sein, dass die Düsen in einem Frontbereich des Patiententischs angeordnet sind. Der Frontbereich des Patiententischs umfasst bevorzugt einen Bereich, der bei einem Einfahren des Patiententischs in den Patientenaufnahmebereich an einem in Fahrtrichtung weisendem Ende des Patiententischs angeordnet ist und/oder der bei einem Einfahren des Patiententischs in den Patientenaufnahmebereich zuerst innerhalb des Patientenaufnahmebereichs einfährt. Dabei kann die Sprüheinheit mit den Düsen in dem Frontbereich des Patiententischs integriert sein oder auch als Zusatzeinheit im Frontbereich des Patiententischs anordbar und/oder positionierbar sein.

Eine derartige Ausgestaltung der Erfindung ermöglicht eine gute Verteilung eines Reinigungsmittels, insbesondere einer Reinigungsflüssigkeit, innerhalb des Patientenaufnahmebereichs, insbesondere an der den Patientenaufnahmebereich umgebenden Umhausung, über eine gesamte Länge des Patientenaufnahmebereichs. Insbesondere kann durch die Anordnung der Düsen im Frontbereich des Patiententischs mit dem Einfahren des Patiententischs in den Patientenaufnahmebereich gleichzeitig mit dem Ausbringen und/oder Versprühen des Reinigungsmittels, insbesondere der Reinigungsflüssigkeit, begonnen werden. Zudem kann derart auch der Patientenaufnahmebereich, insbesondere die den Patientenaufnahmebereich umgebenden Umhausung, entlang einer gesamten Länge, insbesondere von einer Einführöffnung des Patientenaufnahmebereichs bis zu Position des Frontendes des Patiententischs, die das Frontende des Patiententischs bei einem Beenden der Einfahrbewegung des Patiententischs aufweist, gereinigt werden.

In einer vorteilhaften Weiterbildung der erfindungsgemäßen Magnetresonanzvorrichtung kann es vorgesehen sein, dass die Sprüheinheit einen Vorratsbehälter aufweist, der an einer Basiseinheit der Patientenlagerungsvorrichtung angeordnet ist. In diesem Zusammenhang soll unter einer Basiseinheit der Patientenlagerungsvorrichtung eine Einheit verstanden werden, die bei einer horizontalen Bewegung des Patiententisch in den Patientenaufnahmebereich hinein oder aus diesem heraus keine Bewegung gegenüber der Magnetresonanzvorrichtung und/oder gegenüber dem Patientenaufnahmebereich ausführt. Bevorzugt ist die Basiseinheit der Patientenlagerungsvorrichtung auch zu einer Lagerung und/oder einer Abstützung des Patiententischs ausgebildet, wenn sich der Patiententisch außerhalb des Patientenaufnahmebereichs befindet. Der Vorratsbehälter ist bevorzugt zur Aufnahme eines Reinigungsmittels ausgebildet. Insbesondere ist der Vorratsbehälter als Flüssigkeitsbehälter zur Aufnahme einer Reinigungsflüssigkeit ausgebildet. Dabei kann der Vorratsbehälter, insbesondere der Flüssigkeitsbehälter, wiederauffüllbar ausgebildet sein oder auch als austauschbarer und/oder auswechselbarer Einwegbehälter ausgebildet sein. Die Anordnung des Vorratsbehälters, insbesondere des Flüssigkeitsbehälters, innerhalb der Basiseinheit der Patientenlagerungsvorrichtung kann dabei eine Anordnung innerhalb eines Bereichs, der von einem Gehäuse der Basiseinheit umgeben ist, umfassen, wie beispielsweise ein Aufnahmefach zur Aufnahme des Flüssigkeitsbehälters. Alternativ oder zusätzlich kann der Vorratsbehälter, insbesondere der Flüssigkeitsbehälter, auch an einer Außenseite der Basiseinheit der Patientenlagerungsvorrichtung angeordnet sein, wie beispielsweise eine Anordnung mittels eines Halteelements und/oder eines Befestigungselements an der Basiseinheit. Vorzugsweise ist der Vorratsbehälter, insbesondere der Flüssigkeitsbehälter, für einen Benutzer und/oder ein Reinigungspersonal zugänglich an der Basiseinheit angeordnet.

Eine derartige Ausgestaltung der Erfindung weist den Vorteil auf, dass ein genügend großer Vorrat an Reinigungsmittel, insbesondere an Reinigungsflüssigkeit, für eine Reinigung der den Patientenaufnahmebereich umgebenden Umhausung vorhanden ist und die Reinigung der den Patientenaufnahmebereich umgebenden Umhausung ohne Unterbrechung durchgeführt werden kann. Zudem kann die Sprüheinheit auch mehr als einen Vorratsbehälter, insbesondere mehr als einen Flüssigkeitsbehälter, aufweisen, wobei in den zwei oder mehr Vorratsbehältern unterschiedliche Reinigungsmittel für eine Reinigung mit unterschiedlichen Reinigungsstufen der den Patientenaufnahmebereich umgebenden Umhausung aufweisen.

Alternativ hierzu kann bei einer Ausbildung der Reinigungseinheit, insbesondere der Sprüheinheit, als eine am Patiententisch anbringbare und/oder abnehmbare Zusatzeinheit auch der Vorratsbehälter zur Aufnahme des Reinigungsmittels auch als Zusatzeinheit ausgebildet sein. Für einen Reinigungsvorgang der den Patientenaufnahmebereich umgebenden Umhausung kann dabei auch der als Zusatzeinheit ausgebildete Vorratsbehälter auf dem Patiententisch anordbar und/oder positionierbar sein. Zudem kann es auch sein, dass für einen Reinigungsvorgang der den Patientenaufnahmebereich umgebenden Umhausung der als Zusatzeinheit ausgebildete Vorratsbehälter auch an der Basiseinheit anordbar und/oder positionierbar ist, wie beispielsweise mittels Befestigungsmittel und/oder Haltemittel, und/oder an weiteren, den Fachmann als sinnvoll erscheinenden Orten während einer Reinigung der den Patientenaufnahmebereich umgebenden Umhausung positioniert werden.

Die Sprüheinheit kann zudem weitere Einheiten aufweisen, wie beispielsweise eine Pumpe und/oder einen Reinigungsmittelkanal usw. Der Vorratsbehälter, insbesondere der Flüssigkeitsbehälter, ist bevorzugt mittels des Reinigungsmittelkanals den Düsen der Sprüheinheit verbunden, so dass von dem Vorratsbehälter, insbesondere dem Flüssigkeitsbehälter, das Reinigungsmittel zu den Düsen geführt werden kann. Das Reinigungsmittels, insbesondere die Reinigungsflüssigkeit, wird bevorzugt mittels der Pumpe von dem Vorratsbehälter zu den Düsen gepumpt. Zudem kann die Sprüheinheit und/oder die Reinigungseinheit auch eine Steuerungseinheit umfassen, die ein Verteilen und/oder ein Ausbringen des Reinigungsmittels, insbesondere ein Versprühen der Reinigungsflüssigkeit, mittels der Sprüheinheit steuert.

In einer vorteilhaften Weiterbildung der erfindungsgemäßen Magnetresonanzvorrichtung kann es vorgesehen sein, dass die Reinigungseinheit eine Trocknungseinheit aufweist, wobei die Trocknungseinheit zu einem Einströmen einer Trocknungsluft in den Patientenaufnahmebereich ausgebildet ist. Die Trocknungseinheit umfasst hierzu bevorzugt Lufteinlassöffnungen, die in den Patientenaufnahmebereich münden. Diese Lufteinlassöffnungen können beispielsweise Düsen umfassen, durch die die Trocknungsluft in den Patientenaufnahmebereich einströmen kann. Diese Düsen können speziell zum Einströmen von Luft, insbesondere von Trocknungsluft, ausgebildet sein. Zudem können diese Düsen der Trocknungseinheit auch zumindest teilweise einstückig und/oder einteilig mit den Düsen der Saugeinheit ausgebildet sein, so dass eine besonders kompakte Reinigungseinheit zur Reinigung des Patientenaufnahmebereichs, insbesondere des den Patientenaufnahmebereichs umgebenden Umhausung, erreicht werden kann. Bevorzugt weist die Trocknungseinheit des Weiteren einen Lüfter und/oder Ventilator auf zur Erzeugung eines Luftstroms und einen Luftzufuhrkanal zu einem Transport der Trocknungsluft.

Diese Ausgestaltung der Erfindung weist den Vorteil auf, dass eine schnelle Trocknung des Patientenaufnahmebereichs, insbesondere der den Patientenaufnahmebereich umgebenden Umhausung, nach einem Reinigungsvorgang bereitgestellt wird und damit eine lange Wartezeit verhindert werden kann bis die Magnetresonanzvorrichtung wieder für eine Magnetresonanzuntersuchung zur Verfügung steht. Damit einhergehend kann trotz einer Reinigung des Patientenaufnahmebereichs nach jeder Magnetresonanzuntersuchung und/oder nach jedem Patientenwechsel eine hohe Anzahl an Magnetresonanzuntersuchungen und damit eine hohe Auslastung der Magnetresonanzvorrichtung erreicht werden.

In einer vorteilhaften Weiterbildung der erfindungsgemäßen Magnetresonanzvorrichtung kann es vorgesehen sein, dass die Trocknungseinheit zumindest teilweise am Patiententisch und/oder an der den Patientenaufnahmebereich umgebenden Umhausung angeordnet ist. Bei einer Anordnung der Trocknungseinheit am Patiententisch kann die Trocknungseinheit zumindest teilweise innerhalb des Patiententischs integriert sein. Zudem kann hierbei die Trocknungseinheit auch als Zusatzeinheit für eine Reinigung des Patientenaufnahmebereichs auf dem Patiententisch positioniert werden. Derart kann insbesondere ein Reinigungsvorgang der den Patientenaufnahmebereichs umgebenden Umhausung optimiert werden, indem beispielsweise bei einem Einfahren des Patiententischs in den Patientenaufnahmebereich das Reinigungsmittel, insbesondere die Reinigungsflüssigkeit, mittels der Sprüheinheit, insbesondere die Düsen der Sprüheinheit, an die Umhausung des Patientenaufnahmebereichs verteilt wird und bei einem Ausfahren des Patiententischs aus dem Patientenaufnahmebereich die Trocknungseinheit eine Trocknung der den Patientenaufnahmebereich umgebenden Umhausung bewirken.

Alternativ oder zusätzlich kann die Trocknungseinheit zumindest teilweise auch an der den Patientenaufnahmebereich umgebenden Umhausung angeordnet sein. Dabei kann die Trocknungseinheit beispielsweise innerhalb eines Patientenbelüftungssystems, das zu einer Belüftung des Patientenaufnahmebereichs ausgebildet ist, integriert sein. Derart kann eines besonders kompakte und Bauraum sparende Reinigungseinheit bereitgestellt werden.

In einer vorteilhaften Weiterbildung der erfindungsgemäßen Magnetresonanzvorrichtung kann es vorgesehen sein, dass die Reinigungseinheit eine Absaugeinheit aufweist. Durch eine Absaugeinheit kann vorteilhaft ein MAK(Maximale Arbeitsplatzkonzentration)-Wert von Reinigungsmittelaerosolen in der Luft nach dem Auftragen des Reinigungsmittels, insbesondere einem Versprühen der Reinigungsflüssigkeit, in einem zulässigen Bereich gehalten werden und dadurch auch eine Gefährdung und/oder eine gesundheitliche Beeinträchtigung des Patienten bei einer der Reinigung der den Patientenaufnahmebereich umgebenden Umhausung nachfolgenden Magnetresonanzuntersuchung reduziert werden.

Die Absaugeinheit umfasst bevorzugt einen Ventilator und/oder einen Lüfter und/oder eine Absaugpumpe, der und/oder die zu einer Generierung eines Saugstroms ausgebildet ist. Des Weiteren weist die Absaugeinheit bevorzugt zumindest eine Absaugöffnung auf, die beispielsweise auch eine Absaugdüse umfassen kann. Zudem kann die Absaugeinheit auch einen Absaugkanal umfassen, der eine angesaugte Luft weitertransportiert, insbesondere aus dem Patientenaufnahmebereich wegtransportiert.

In einer vorteilhaften Weiterbildung der erfindungsgemäßen Magnetresonanzvorrichtung kann es vorgesehen sein, dass die Absaugeinheit zumindest teilweise an der den Patientenaufnahmebereich umgebenden Umhausung und/oder am Patiententisch angeordnet ist. Vorzugsweise weist die Absaugeinheit zumindest eine Absaugöffnung und/oder zumindest eine Saugdüse auf, wobei die zumindest eine Absaugöffnung und/oder die zumindest eine Saugdüse an der den Patientenaufnahmebereich umgebenden Umhausung und/oder am Patiententisch angeordnet ist. Die zumindest eine Absaugöffnung und/oder die zumindest eine Saugdüse der Absaugeinheit kann dabei zumindest teilweise innerhalb des Patiententischs integriert sein. Die Absaugeinheit, insbesondere die zumindest eine Absaugöffnung und/oder die zumindest eine Saugdüse der Absaugeinheit, kann zudem auch zumindest teilweise einstückig und/oder einteilig mit der Sprüheinheit ausgebildet sein. Beispielsweise können hierbei die Düsen der Sprüheinheit als Doppeldüse ausgebildet sein, die sowohl ein Reinigungsmittel ausbringen und/oder versprühen können, als auch nach dem Ausbringen des Reinigungsmittels zu einem Absaugen von Reinigungsaerosolen ausgebildet sein können.

Zudem kann die Absaugeinheit auch als Zusatzeinheit auf dem Patiententisch für eine Reinigung der den Patientenaufnahmebereich umgebenden Umhausung angeordnet und/oder positioniert werden. Dabei kann die Absaugeinheit in Einfahrrichtung des Patiententischs beispielsweise nach der Sprüheinheit angeordnet sein, insbesondere in einem mittigen Bereich des Patiententischs und/oder in einem hinteren Bereich des Patiententischs angeordnet sein. Zudem kann die Absaugeinheit auch eine Absaugöffnung aufweisen, die an der Umhausung des Patientenaufnahmebereichs angeordnet ist und somit die Luft aus dem Patientenaufnahmebereich absaugen kann. Die Absaugeinheit kann dabei eine oder mehrere Absaugöffnungen und/oder Saugdüsen aufweisen.

Durch diese Ausgestaltung der Erfindung kann der Vorteil erreicht werden, dass eine besonders platzsparende Anordnung der Absaugeinheit bereitgestellt werden kann.

In einer vorteilhaften Weiterbildung der erfindungsgemäßen Magnetresonanzvorrichtung kann es vorgesehen sein, dass die Absaugeinheit zumindest einen Absaugkanal mit einem Filter umfasst. Derart kann die Luft aus dem Patientenaufnahmebereich nicht nur abgesaugt werden sondern auch hinsichtlich von Reinigungsaerosolen gereinigt werden. Zudem kann derart besonders effizient ein MAK-Wert des Reinigungsmittels eingehalten werden. Vorzugsweise weist der Lüftungskanal auch eine Auslassöffnung auf, wobei der Filter in einer Strömungsrichtung einer angesaugten Luft vor der Auslassöffnung angeordnet ist. Der Filter ist bevorzugt auswechselbar innerhalb des Absaugkanals angeordnet, wobei hierzu der Filter in der Nähe der Auslassöffnung angeordnet ist, um somit eine leichte Zugänglichkeit für einen Filterwechsel für einen Benutzer, beispielsweise ein Reinigungspersonal, zu ermöglichen.

Des Weiteren geht die Erfindung aus von einem Verfahren zu einem Reinigen eines Patientenaufnahmebereichs, insbesondere einer den Patientenaufnahmebereich umgebenden Umhausung, einer Magnetresonanzvorrichtung mittels einer Reinigungseinheit, wobei das Verfahren die folgenden Schritte umfasst:
- Einfahren eines Patiententischs in den Patientenaufnahmebereich, wobei an dem Patiententisch die Reinigungseinheit angeordnet ist,
- Gleichzeitig mit dem Einfahren des Patiententischs in den Patientenaufnahmebereich erfolgt ein Ausführen eines ersten Reinigungsschritts eines Reinigungsvorgangs, wobei der erste Reinigungsschritt ein Ausbringen eines Reinigungsmittels mittels der Reinigungseinheit umfasst, und
- Ausführen eines zweiten Reinigungsschritts, wobei der zweite Reinigungsschritt einen Trocknungsschritt und/oder einen Absaugschritt umfasst.

Das Ausbringen des Reinigungsmittels umfasst bevorzugt ein Verteilen, insbesondere ein Versprühen, einer Reinigungsflüssigkeit mittels einer Sprüheinheit der Reinigungseinheit. Die Sprüheinheit weist bevorzugt zumindest eine Düse auf, die innerhalb des Patiententischs integriert sein kann. Zudem kann die Sprüheinheit auch als Zusatzeinheit auf dem Patiententisch für einen Reinigungsvorgang angeordnet und/oder positioniert werden. Bevorzugt ist die Sprüheinheit, insbesondere die zumindest eine Düse der Sprüheinheit, in einem Frontbereich des Patiententischs angeordnet und/oder positioniert.

Aufgrund der Anordnung und/oder Positionierung der Reinigungseinheit, insbesondere der Sprüheinheit, im Frontbereich des Patiententischs befindet sich die Sprüheinheit bereits zu Beginn des Einfahrens des Patiententischs in den Patientenaufnahmebereichs innerhalb des Patientenaufnahmebereichs. Daher kann auch mit dem Einfahren des Patiententischs in den Patientenaufnahmebereich auch gleichzeitig mit dem Ausbringen des Reinigungsmittels, insbesondere einem Versprühen der Reinigungsflüssigkeit, mittels der Sprüheinheit begonnen werden. Hierdurch kann auch der Patientenaufnahmebereich, insbesondere die den Patientenaufnahmebereich umgebenden Umhausung, entlang ihrer gesamten Länge mit einem Reinigungsmittels besprüht und damit gereinigt werden.

Der zweite Reinigungsschritt des Reinigungsvorgangs umfasst den Trocknungsschritt und/oder den Absaugschritt. Sowohl der Trocknungsschritt als auch der Absaugschritt werden mittels der Reinigungseinheit ausgeführt. Hierzu weist die Reinigungseinheit bevorzugt eine Trocknungseinheit und/oder eine Absaugeinheit auf. Mittels der Trocknungseinheit kann eine Trocknungsluft in den Patientenaufnahmebereich eingeblasen werden, die eine rasche Trocknung der Oberfläche des Patientenaufnahmebereichs, insbesondere der Oberfläche der Umhausung des Patientenaufnahmebereichs, bewirkt. Mittels der Absaugeinheit können Reinigungsmittelaerosole abgesaugt werden und damit eine Konzentration der Reinigungsmittelaerosole geringgehalten werden, insbesondere ein MAK-Wert der Reinigungsmittelaerosole eingehalten werden. Vorzugsweise ist die Trocknungseinheit und/oder die Absaugeinheit hierbei an dem Patiententisch angeordnet und/oder an der den Patientenaufnahmebereich umgebenden Umhausung angeordnet. Bei einer Anordnung der Trocknungseinheit und/oder der Absaugeinheit an dem Patiententisch kann dabei die Trocknungseinheit und/oder die Absaugeinheit zumindest teilweise in dem Patiententisch integriert sein. Alternativ hierzu kann es auch sein, dass die Trocknungseinheit und/oder die Absaugeinheit als Zusatzeinheit auf dem Patiententisch für einen Reinigungsvorgang zur Reinigung der den Patientenaufnahmebereich umgebenden Umhausung anordbar und/oder positionierbar sind.

Das erfindungsgemäße Verfahren zum Reinigen des Patientenaufnahmebereichs, insbesondere der den Patientenaufnahmebereich umgebenden Umhausung, ermöglicht eine einfache und effiziente Reinigung der den Patientenaufnahmebereich umgebenden Umhausung der Magnetresonanzvorrichtung. Bevorzugt kann hierbei der Reinigungsvorgang mittels der Reinigungseinheit während eines einmaligen Ein- und Ausfahrens des Patiententischs durchgeführt werden und damit eine besonders zeitsparende Reinigung der den Patientenaufnahmebereich umgebenden Umhausung ermöglicht werden.

Die Vorteile des erfindungsgemäßen Verfahrens zu einem Reinigen einer den Patientenaufnahmebereich umgebenden Umhausung entsprechen im Wesentlichen den Vorteilen der erfindungsgemäßen Magnetresonanzvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

In einer vorteilhaften Weiterbildung des erfindungsgemäßen Verfahrens zu einem Reinigen eines Patientenaufnahmebereichs kann es vorgesehen sein, dass der zweite Reinigungsschritt während des Einfahrens des Patiententischs in den Patientenaufnahmebereich und/oder während eines Ausfahrens des Patiententischs aus dem Patientenaufnahmebereich ausgeführt wird. Dabei kann der zweite Reinigungsschritt bereits mit dem ersten Einfahren des Patiententischs in den Patientenaufnahmebereichs, bei dem auch der erste Reinigungsschritt ausgeführt wird, ausgeführt werden. Alternativ oder zusätzlich kann der zweite Reinigungsschritt auch beim Ausfahren des Patiententischs aus dem Patientenaufnahmebereich ausgeführt werden, so dass zur Reinigung des Patientenaufnahmebereichs der Patiententisch nur ein einziges Mal in Patientenaufnahmebereich eingeführt und ausgeführt werden muss. Derart kann eine besonders schnelle und zeitsparende Reinigung des Patientenaufnahmebereichs ermöglicht werden.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanzvorrichtung mit einer Reinigungseinheit in einer schematischen Darstellung,
- Fig. 2: eine vereinfachte Darstellung der Magnetresonanzvorrichtung mit einer alternativen Ausgestaltung einer Reinigungseinheit,
- Fig. 3: eine vereinfachte Darstellung der Magnetresonanzvorrichtung mit einer weiteren alternativen Ausgestaltung einer Reinigungseinheit,
- Fig. 4: eine vereinfachte Darstellung der Magnetresonanzvorrichtung mit einer weiteren alternativen Ausgestaltung einer Reinigungseinheit,
- Fig. 5: eine vereinfachte Darstellung der Magnetresonanzvorrichtung mit einer weiteren alternativen Ausgestaltung einer Reinigungseinheit und
- Fig. 6: ein erfindungsgemäßes Verfahren zu einem Reinigen eines Patientenaufnahmebereichs einer Magnetresonanzvorrichtung.

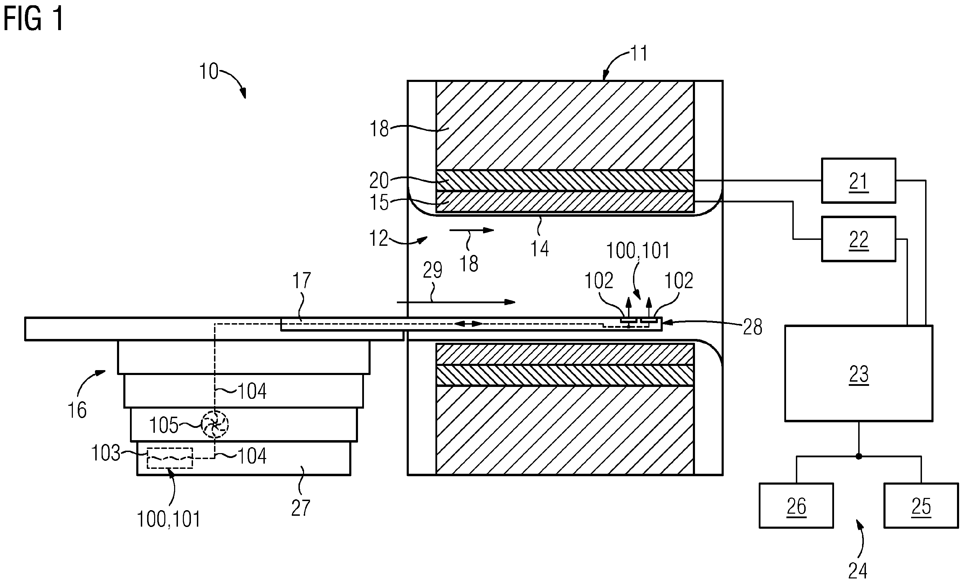

In der Fig. 1 ist eine Magnetresonanzvorrichtung 10 schematisch darstellt. Die Magnetresonanzvorrichtung 10 umfasst eine von einer Magneteinheit gebildeten Scannereinheit 11. Zudem weist die Magnetresonanzvorrichtung 10 einen Patientenaufnahmebereich 12 auf zu einer Aufnahme eines Patienten. Der Patientenaufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Patientenaufnahmebereichs 12 jederzeit denkbar.

Die Scannereinheit 11 weist dabei eine den Patientenaufnahmebereich 12 umgebende Umhausung 14 auf. Die den Patientenaufnahmebereich 12 umgebende Umhausung 14 kann dabei einstückig mit einer Hochfrequenzantenneneinheit 15 der Scannereinheit 11, insbesondere der Magneteinheit, ausgebildet sein. Zudem kann die den Patientenaufnahmebereich 12 umgebende Umhausung 14 auch separat zur Hochfrequenzantenneneinheit 15 ausgebildet sein.

Der Patient kann mittels einer Patientenlagerungsvorrichtung 16 der Magnetresonanzvorrichtung 10 in den Patientenaufnahmebereich 12 geschoben und/oder gefahren werden. Die Patientenlagerungsvorrichtung 16 weist hierzu einen innerhalb des Patientenaufnahmebereichs 12 bewegbar ausgestalteten Patiententisch 17 auf. Insbesondere ist hierbei der Patiententisch 17 in Richtung einer Längserstreckung des Patientenaufnahmebereichs 12 und/oder in horizontaler Richtung bewegbar gelagert.

Die Scannereinheit 11, insbesondere die Magneteinheit, umfasst einen supraleitenden Grundmagneten 18 zu einem Erzeugen eines starken und insbesondere konstanten Grundmagnetfelds 19. Weiterhin weist die Scannereinheit 11, insbesondere die Magneteinheit, eine Gradientenspuleneinheit 20 zu einer Erzeugung von Magnetfeldgradienten auf, die für eine Ortskodierung während einer Bildgebung verwendet werden. Die Gradientenspuleneinheit 20 wird mittels einer Gradientensteuereinheit 21 der Magnetresonanzvorrichtung 10 gesteuert. Die Scannereinheit 11, insbesondere die Magneteinheit, umfasst weiterhin die Hochfrequenzantenneneinheit 15 zu einer Anregung einer Polarisation, die sich in dem von dem Grundmagneten 18 erzeugten Grundmagnetfeld 19 einstellt. Die Hochfrequenzantenneneinheit 15 wird von einer Hochfrequenzantennensteuereinheit 22 der Magnetresonanzvorrichtung 10 gesteuert und strahlt hochfrequente Magnetresonanzsequenzen in den Patientenaufnahmebereich 12 der Magnetresonanzvorrichtung 10 ein.

Zu einer Steuerung des Grundmagneten 18, der Gradientensteuereinheit 21 und zur Steuerung der Hochfrequenzantennensteuereinheit 22 weist die Magnetresonanzvorrichtung 10 eine Systemsteuereinheit 23 auf. Die Systemsteuereinheit 23 steuert zentral die Magnetresonanzvorrichtung, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Anzeigeeinheit 25, beispielsweise auf zumindest einem Monitor, der Benutzerschnittstelle 24 für ein medizinisches Bedienpersonal angezeigt werden. Weiterhin weist die Benutzerschnittstelle 24 eine Eingabeeinheit 26 auf, mittels der Informationen und/oder Parameter während eines Messvorgangs von dem medizinischen Bedienpersonal eingegeben werden können.

Zu einer Reinigung des Patientenaufnahmebereichs 12, insbesondere der den Patientenaufnahmebereich 12 umgebende Umhausung 14 der Scannereinheit 11, weist die Magnetresonanzvorrichtung 10 eine Reinigungseinheit 100 auf. Die Reinigungseinheit 100 ist zumindest teilweise am Patiententisch 17 der Patientenlagerungsvorrichtung 16 angeordnet. Hierbei sind einzelne Teile und/oder Einheiten der Reinigungseinheit 100 am Patiententisch 17 angeordnet. Des Weiteren sind auch einzelne Teile und/oder Einheiten der Reinigungseinheit 100 an der Patientenlagerungsvorrichtung 16, insbesondere an einer Basiseinheit 27 der Patientenlagerungsvorrichtung 16, angeordnet. Die Reinigungseinheit 100 umfasst dabei zumindest eine Einheit, die innerhalb des Patiententischs 17 angeordnet und/oder integriert ist. Mit anderen Worten ist hierbei die zumindest eine Einheit innerhalb eines von einem Gehäuse des Patiententischs 17 umschlossenen Bereichs angeordnet.

Die Reinigungseinheit 100 weist eine Sprüheinheit 101 auf, die zu einem Ausbringen eines Reinigungsmittels ausgebildet ist. Die Sprüheinheit 101 weist hierbei zumindest eine Düse 102 auf, die zum Ausbringen des Reinigungsmittels, insbesondere einem Versprühen einer Reinigungsflüssigkeit, ausgebildet sind. Bevorzugt weist hierbei die Sprüheinheit 101 zwei oder mehr Düsen 102 auf, so dass ein gezieltes Verteilen des Reinigungsmittels, insbesondere ein gezieltes Versprühen der Reinigungsflüssigkeit, an die den Patientenaufnahmebereich 12 umgebende Umhausung 14 bereitgestellt werden kann. Die zumindest eine Einheit der Reinigungseinheit 100, die innerhalb des Patiententischs 17 angeordnet ist, umfasst hierbei die zumindest eine Düse 102 der Sprüheinheit 101. Die zumindest eine Düse 102 der Sprüheinheit 101 ist in einem Frontbereich 28 des Patiententischs 17 angeordnet. Der Frontbereich 28 ist in Einfahrtsrichtung 29 des Patiententischs 17, bei der der Patiententisch 17 in den Patientenaufnahmebereich 12 eingefahren wird, in einem vorderen Bereich angeordnet.

Des Weiteren weist die Sprüheinheit 101 einen Vorratsbehälter 103 auf. Der Vorratsbehälter 103 ist zur Aufnahme des Reinigungsmittels ausgebildet. Der Vorratsbehälter 103 ist an der Basiseinheit 27 der Patientenlagerungsvorrichtung 16 angeordnet. Dabei ist der Vorratsbehälter 103 derart an der Basiseinheit 27 angeordnet, dass er für einen Austausch des Vorratsbehälters 103 und/oder für ein Wiederauffüllen des Vorratsbehälters 103 für einen Bediener, insbesondere ein medizinisches Reinigungspersonal erreichbar ist. Die Basiseinheit 27 kann dabei ein von außen zugängliches Fach zur Aufnahme des Vorratsbehälters 103 umfassen.

Die Reinigungseinheit 100, insbesondere die Sprüheinheit 101, weist zudem einen Reinigungsmittelkanal 104 und/oder eine Reinigungsmittelleitung auf, wobei der Reinigungsmittelkanal 104 und/oder die Reinigungsmittelleitung zu einem Transport des Reinigungsmittel zwischen dem Vorratsbehälter 103 und der zumindest einen Düse 102 innerhalb der Patientenlagerungsvorrichtung 16 angeordnet ist. Der Reinigungsmittelkanal 104 und/oder die Reinigungsmittelleitung kann beispielsweise einen Schlauch und/oder weitere dem Fachmann als sinnvoll erscheinende Zuleitungen aufweisen. Zudem weist die Reinigungseinheit 100, insbesondere die Sprüheinheit 101, eine Pumpe 105 auf zu einem Transport und/oder einer Beförderung des Reinigungsmittels innerhalb des Reinigungsmittelkanals 104 und/oder innerhalb der Reinigungsmittelleitung. Die Pumpe 105 ist ebenfalls innerhalb der Basiseinheit 27 der Patientenlagerungsvorrichtung 16 angeordnet.

Die Reinigungseinheit 100, insbesondere die Sprüheinheit 101, wird dabei für eine Reinigungsvorgang aktiviert, während der Patiententisch 17 in den Patientenaufnahmebereich 12 einfährt, so dass der Patientenaufnahmebereich 12, insbesondere den Patientenaufnahmebereich 12 umgebende Umhausung 14, entlang einer gesamten Länge des Patientenaufnahmebereichs 12 mit dem Reinigungsmittel besprüht wird.

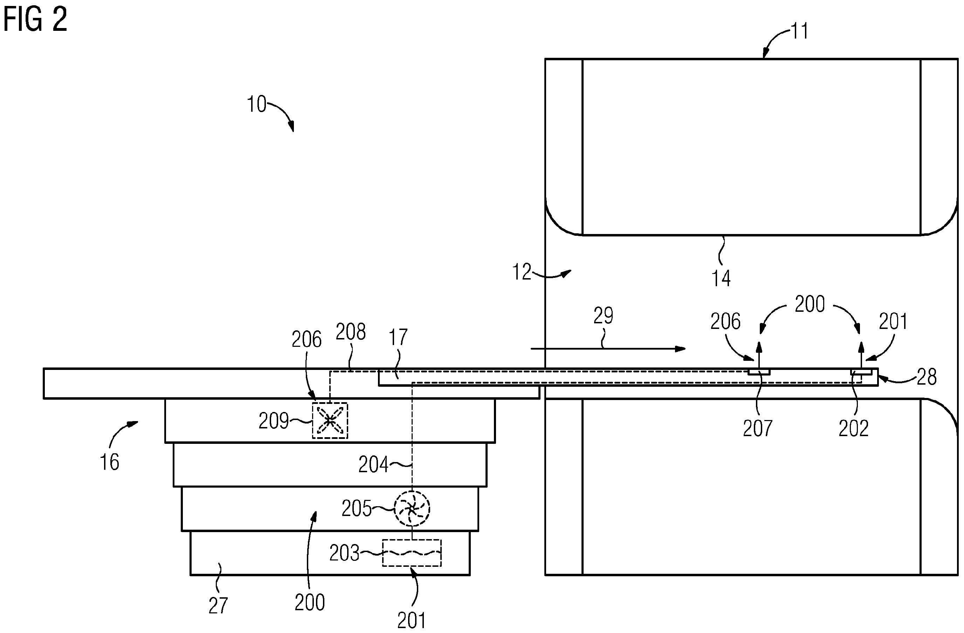

In Fig. 2 ist ein alternatives Ausführungsbeispiel einer Reinigungseinheit 200 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 1, wobei bezüglich gleichbleibender Bauteile, Merkmale und Funktionen auf die Beschreibung des Ausführungsbeispiels in Fig. 1 verwiesen wird.

Die Reinigungseinheit 200 in Fig. 2 ist ebenfalls zur Reinigung der den Patientenaufnahmebereich 12 umgebenden Umhausung 14 ausgebildet und zumindest teilweise am Patiententisch 17 angeordnet. Die Reinigungseinheit 200 weist hierbei eine Sprüheinheit 201 mit zumindest einer Düse 202, einem Vorratsbehälter 203, einem Reinigungsmittelkanal 204 und einer Pumpe 205 auf. Bezüglich einer Anordnung und einer Ausgestaltung der Sprüheinheit 201 auf die Ausführungen zu Fig. 1 verwiesen wird.

Die Reinigungseinheit 200 weist des Weiteren eine Trocknungseinheit 206 auf. Die Trocknungseinheit 206 ist zu einem Einströmen einer Trocknungsluft in den Patientenaufnahmebereich 12 ausgebildet. Die Trocknungseinheit 206 ist im vorliegenden Ausführungsbeispiel zumindest teilweise innerhalb des Patiententischs 17 angeordnet. Insbesondere weist hierbei die Trocknungseinheit 206 zumindest eine Luftdüse 207 auf, die innerhalb des Patiententischs 17 angeordnet sind. Die Trocknungseinheit 206 weist des Weiteren einen Lüftungskanal 208 und einen Lüfter 209 und/oder Ventilator auf. Der Ventilator und/oder Lüfter 209 ist zur Generierung eines Luftstrom ausgebildet. Zudem sind der Lüfter 209 und/oder Ventilator innerhalb der Basiseinheit 27 angeordnet. Der Lüftungskanal 208 ist zu einer Luftzufuhr in den Patientenaufnahmebereich 12, insbesondere von dem Lüfter 209 und/oder Ventilator zur der zumindest einen Luftdüse 207 ausgebildet. Zudem kann die Trocknungseinheit 206 auch noch eine nicht näher dargestellt Luftansaugöffnung aufweisen, die an einer Außenseite der Basiseinheit 27 angeordnet ist.

Für einen Reinigungsvorgang kann hierbei die Sprüheinheit 201 der Reinigungseinheit 200 während eines Einfahrens des Patiententischs 17 in den Patientenaufnahmebereich 12 aktiviert werden, so dass der Patientenaufnahmebereich 12, insbesondere die des Patientenaufnahmebereich 12 umgebende Umhausung 14, entlang einer gesamten Länge des Patientenaufnahmebereichs 12 mit dem Reinigungsmittel besprüht wird. Bei einem Ausfahren des Patiententisch 17 aus dem Patientenaufnahmebereichs 12 kann dagegen die Trocknungseinheit 206 der Reinigungseinheit 200 aktiviert werden und derart der Patientenaufnahmebereich 12, insbesondere die den Patientenaufnahmebereich 12 umgebende Umhausung 14, getrocknet werden.

In einer alternativen Ausgestaltung der Trocknungseinheit 206 kann die zumindest eine Luftdüse 207 auch einstückig und/oder einteilig mit der zumindest einen Düse 202 der Saugeinheit 201 ausgebildet sein. Hierbei könnte die zumindest eine Düse 202, 207 in einem ersten Betriebsmodus ein Verteilen des Reinigungsmittels ausführen und in einem zweiten Betriebsmodus einen Luftstrom in den Patientenaufnahmebereich 12 einführen. In einer weiteren alternativen Ausgestaltung der Trocknungseinheit 206 kann die zumindest eine Luftdüse 207 auch in der den Patientenaufnahmebereich 12 umgebenden Umhausung 14 angeordnet sein. Bei einer derartigen Ausgestaltung der Trocknungseinheit 206 weist die Umhausung 14 eine Lüftungsöffnung auf, durch die eine Trocknungsluft in den Patientenaufnahmebereich 12 einströmen kann. In einer weiteren alternativen Ausgestaltung der Trocknungseinheit 206 kann diese auch einteilig und/oder einstückig mit einer nicht näher dargestellten Patientenbelüftungseinheit ausgebildet sein. Die Patientenbelüftungseinheit weist bevorzugt eine Belüftungsöffnung auf, die in der den Patientenaufnahmebereichs 12 umgebenden Umhausung 14 angeordnet ist.

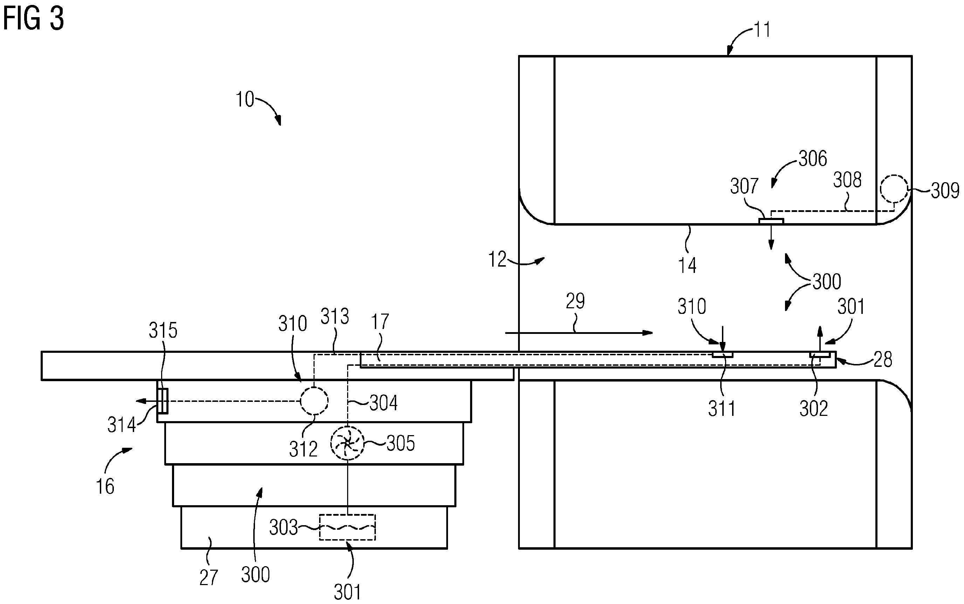

In Fig. 3 ist ein alternatives Ausführungsbeispiel einer Reinigungseinheit 300 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 und 2, wobei bezüglich gleichbleibender Bauteile, Merkmale und Funktionen auf die Beschreibung der Ausführungsbeispiele in den Fig. 1 und 2 verwiesen wird.

Die Reinigungseinheit 300 in Fig. 3 ist ebenfalls zur Reinigung der den Patientenaufnahmebereich 12 umgebenden Umhausung 14 ausgebildet und zumindest teilweise am Patiententisch 17 angeordnet. Die Reinigungseinheit 300 weist hierbei eine Sprüheinheit 301 mit zumindest einer Düse 302, einem Vorratsbehälter 303, einem Reinigungsmittelkanal 304 und einer Pumpe 305 auf. Bezüglich einer Anordnung und einer Ausgestaltung der Sprüheinheit 301 auf die Ausführungen zu den Fig. 1 und 2 verwiesen wird.

Die Reinigungseinheit 300 weist zudem eine Trocknungseinheit 306 auf, wobei die Trocknungseinheit 306 im vorliegenden Ausführungsbeispiel beispielhaft an der den Patientenaufnahmebereich 12 umgebenden Umhausung 14 angeordnet ist. Die Trocknungseinheit 306 weist hierbei eine Luftdüse 307, einen Lüftungskanal 308 und einen Lüfter 309 und/oder Ventilator auf, die innerhalb eines die Scannereinheit 11 umgebenden Gehäuses angeordnet sind. Eine Funktionsweise der Trocknungseinheit 306 entspricht ist im Wesentlichen gleich zu einer Funktionsweise der Trocknungseinheit 206 in Fig. 2 ausgebildet. Alternativ hierzu könnte die Trocknungseinheit 306 auch wie in Fig. 2 beschrieben ausgebildet sein.

Die Reinigungseinheit 300 weist des Weiteren eine Absaugeinheit 310 auf. Mittels der Absaugeinheit 310 kann ein Absaugen eines Reinigungsmittelaerosols nach einem Ausbringen des Reinigungsmittels aus dem Patientenaufnahmebereich 12 erfolgen. Die Absaugeinheit 310 ist im vorliegenden Ausführungsbeispiel zumindest teilweise am Patiententisch 17 angeordnet. Hierzu weist die Absaugeinheit 310 zumindest eine Saugdüse 311 auf, die am Patiententisch 17 angeordnet ist. Die zumindest eine Saugdüse 311 in Richtung einer Längserstreckung des Patiententischs 17 nach der Düse 302 der Saugeinheit 301 angeordnet. Die Absaugeinheit 310 umfasst weiterhin einen Lüfter 312 und/oder Ventilator und/oder eine Absaugpumpe zur Erzeugung eines Luftstroms, insbesondere eines Saugstroms, der ein Absaugen von Luft und/oder Reinigungsmittelaerosolen aus dem Patientenaufnahmebereich 12 bewirkt. Des Weiteren umfasst die Absaugeinheit 310 einen Absaugkanal 313, der zu einem Ableiten der angesaugten Luft und/oder Reinigungsmittelaerosolen aus dem Patientenaufnahmebereich 12 von der zumindest einen Saugdüse 311 zu einer Ausblasöffnung 314 der Absaugeinheit 310. Die Ausblasöffnung 314 ist an einem Gehäuse der Basiseinheit 27 der Patientenlagerungsvorrichtung 16 angeordnet. Der Lüfter 312 und/oder Ventilator und/oder die Absaugpumpe ist zudem innerhalb des Absaugkanals 313 angeordnet. Die Absaugeinheit 310 weist weiterhin einen Filter 315 auf, der zur Reinigung der angesaugten Luft mit den Reinigungsmittelaerosolen ausgelegt ist. Der Filter 315 ist innerhalb des Absaugkanals 313 angeordnet, wobei der Filter 315 im Bereich der Auslassöffnung 314 innerhalb des Absaugkanals 313 angeordnet ist.

In einer alternativen Ausgestaltung der Reinigungseinheit 200, insbesondere der Absaugeinheit 310, kann diese auch zumindest teilweise einstückig und/oder einteilig mit der Sprüheinheit 301 ausgebildet sein. Hierbei kann insbesondere die zumindest eine Saugdüse 311 der Absaugeinheit 310 einstückig und/oder einteilig mit der Düse 302 der Sprüheinheit 301 ausgebildet sein. Die zumindest eine Düse 302 kann dabei eine Art Doppeldüse umfassen, die sowohl zu einem Verteilen des Reinigungsmittels, insbesondere einem Versprühen der Reinigungsflüssigkeit, als auch zu einem Absaugen von Reinigungsmittelaerosolen ausgebildet ist.

Für einen Reinigungsvorgang kann hierbei die Sprüheinheit 301 der Reinigungseinheit 300 während eines Einfahrens des Patiententischs 17 in den Patientenaufnahmebereich 12 aktiviert werden, so dass der Patientenaufnahmebereich 12, insbesondere die den Patientenaufnahmebereich 12 umgebende Umhausung 14, entlang einer gesamten Länge des Patientenaufnahmebereichs 12 mit dem Reinigungsmittel besprüht wird. Dabei kann gleichzeitig mit dem Einfahren des Patiententischs 17 in den Patientenaufnahmebereich 12 oder auch erst bei einem Ausfahren des Patiententischs 17 aus dem Patientenaufnahmebereich 12 die Absaugeinheit 310 aktiviert werden zu einem Absaugen der Reinigungsmittelaerosole aus dem Patientenaufnahmebereich 12. Zudem kann während des Ausfahrens des Patiententischs 17 aus dem Patientenaufnahmebereich 12 die Trocknungseinheit 306 der Reinigungseinheit 300 aktiviert werden und derart der Patientenaufnahmebereich 12, insbesondere die den Patientenaufnahmebereich 12 umgebende Umhausung 14, getrocknet werden.

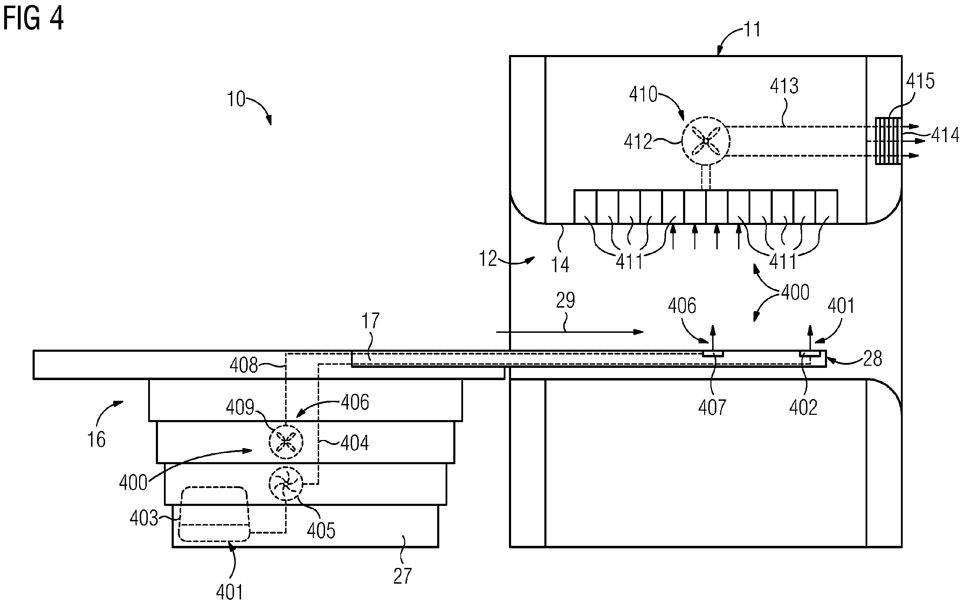

In Fig. 4 ist ein alternatives Ausführungsbeispiel einer Reinigungseinheit 400 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 3, wobei bezüglich gleichbleibender Bauteile, Merkmale und Funktionen auf die Beschreibung der Ausführungsbeispiele in den Fig. 1 bis 3 verwiesen wird.

Die Reinigungseinheit 400 in Fig. 4 ist ebenfalls zur Reinigung der den Patientenaufnahmebereich 12 umgebenden Umhausung 14 ausgebildet und zumindest teilweise am Patiententisch 17 angeordnet. Die Reinigungseinheit 400 weist hierbei eine Sprüheinheit 401 mit zumindest einer Düse 402, einem Vorratsbehälter 403, einem Reinigungsmittelkanal 404 und einer Pumpe 405 auf. Bezüglich einer Anordnung und einer Ausgestaltung der Sprüheinheit 401 wird hierbei auf die Ausführungen zu den Fig. 1 bis 3 verwiesen.

Des Weiteren weist die Reinigungseinheit 400 in Fig. 4 auch eine Trocknungseinheit 406 auf, die zumindest teilweise innerhalb des Patiententischs 17 angeordnet ist. Die Trocknungseinheit 406 weist eine Luftdüse 407, einen Lüftungskanal 408 und einen Lüfter 409 und/oder Ventilator auf. Bezüglich einer Anordnung und einer Ausgestaltung der Trocknungseinheit 406 wird auf die Ausführungen zu Fig. 2 verwiesen.

Die Reinigungseinheit 400 weist weiterhin eine Absaugeinheit 410 auf, mittels der kann ein Absaugen eines Reinigungsmittelaerosols nach einem Ausbringen des Reinigungsmittels aus dem Patientenaufnahmebereich 12 erfolgen kann. Die Absaugeinheit 410 ist zumindest teilweise an der den Patientenaufnahmebereich 12 umgebenden Umhausung 14 angeordnet. Die Absaugeinheit 410 weist zumindest eine Saugdüse 411 auf, die an der den Patientenaufnahmebereich 12 umgebenden Umhausung 14 angeordnet ist. Des Weiteren weist die Absaugeinheit 410 einen Lüfter 412 und/oder Ventilator und/oder eine Absaugpumpe, einen Absaugkanal 413, eine Ausblasöffnung 414 und einen Filter 415 auf. Die Ausblasöffnung 414 ist an einem Gehäuse der Scannereinheit 11, insbesondere an einer Rückseite der Scannereinheit 11, angeordnet. Der Absaugkanal 413 ist innerhalb eines von dem Gehäuse der Scannereinheit 11 umgebenen Bereich angeordnet zu einem Transport einer aus dem Patientenaufnahmebereich 12 abgesaugten Luft und/oder Reinigungsmittelaerosolen zu der Ausblasöffnung 414 ausgebildet. Hinsichtlich einer Funktionsweise der Absaugeinheit 410 wird auf die Ausführung zur Fig. 3 verwiesen.

Für einen Reinigungsvorgang kann hierbei die Sprüheinheit 401 der Reinigungseinheit 400 während eines Einfahrens des Patiententischs 17 in den Patientenaufnahmebereich 12 aktiviert werden, so dass der Patientenaufnahmebereich 12, insbesondere die den Patientenaufnahmebereich 12 umgebende Umhausung 14, entlang einer gesamten Länge des Patientenaufnahmebereichs 12 mit dem Reinigungsmittel besprüht wird. Dabei kann gleichzeitig mit dem Einfahren des Patiententischs 17 in den Patientenaufnahmebereich 12 oder auch erst beim Ausfahren des Patiententischs 17 aus dem Patientenaufnahmebereich 12 die Absaugeinheit 410 aktiviert werden zu einem Absaugen der Reinigungsmittelaerosole aus dem Patientenaufnahmebereich 12. Zudem kann während eines Ausfahrens des Patiententischs 17 aus dem Patientenaufnahmebereich 12 die Trocknungseinheit 406 der Reinigungseinheit 400 aktiviert und derart die den Patientenaufnahmebereich 12 umgebende Umhausung 14 getrocknet werden.

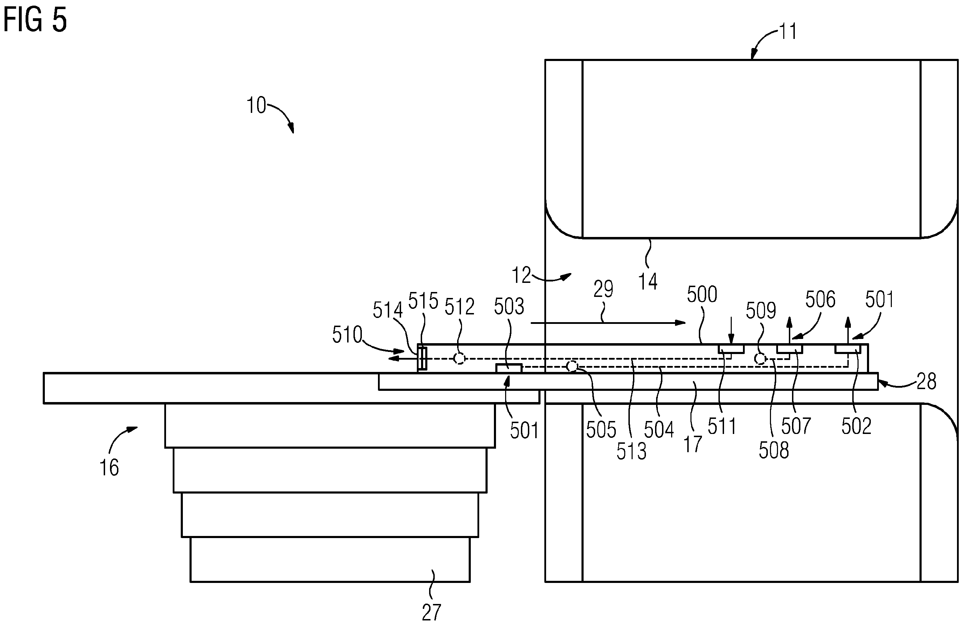

In Fig. 5 ist ein alternatives Ausführungsbeispiel einer Reinigungseinheit 500 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 4, wobei bezüglich gleichbleibender Bauteile, Merkmale und Funktionen auf die Beschreibung der Ausführungsbeispiele in den Fig. 1 bis 4 verwiesen wird.

Die Reinigungseinheit 500 in Fig. 5 ist ebenfalls zur Reinigung der den Patientenaufnahmebereich 12 umgebenden Umhausung 14 ausgebildet und zumindest teilweise am Patiententisch 17 angeordnet. Die Reinigungseinheit 500 ist im vorliegenden Ausführungsbeispiel zumindest teilweise als Zusatzeinheit ausgebildet, wobei die Zusatzeinheit für eine Reinigung der den Patientenaufnahmebereich 12 umgebenden Umhausung 14 auf dem Patiententisch 17 anordbar und/oder positionierbar ist. Die Reinigungseinheit 500 weist hierbei eine Sprüheinheit 501 auf, die von der Zusatzeinheit umfasst ist. Die Sprüheinheit 501 umfasst dabei zumindest eine Düse 502, die zu einem Verteilen des Reinigungsmittels, insbesondere einem Versprühen einer Reinigungsflüssigkeit ausgebildet ist. Die Sprüheinheit 501 weist weiterhin einen Vorratsbehälter 503, einen Reinigungsmittelkanal 504 und eine Pumpe 505 auf, die ebenfalls von der Zusatzeinheit umfasst sind. Die Zusatzeinheit, insbesondere die Sprüheinheit 501, wird für eine Reinigung der den Patientenaufnahmebereich 12 umgebenden Umhausung 14 derart auf dem Patiententisch 17 positioniert und/oder angeordnet, dass die zumindest eine Düse 502 der Sprüheinheit 501 in dem Frontbereich 28 des Patiententischs 17 angeordnet ist. Bezüglich einer Funktionsweise der Sprüheinheit 501 wird auf die Ausführungen zu den Fig. 1 bis 4 verwiesen.

Die Reinigungseinheit 500 weist des Weiteren eine Trocknungseinheit 506 auf, die ebenfalls von der auf dem Patiententisch 17 anordbaren und/oder positionierbaren Zusatzeinheit umfasst ist. Die Trocknungseinheit 506 weist dabei zumindest eine Luftdüse 507, einen Lüftungskanal 508 und einen Lüfter 509 und/oder Ventilator auf. Bezüglich einer Anordnung und einer Ausgestaltung der Trocknungseinheit 506 auf die Ausführungen zu Fig. 3 verwiesen wird.

Die Reinigungseinheit 500 weist des Weiteren eine Absaugeinheit 510 auf, die ebenfalls von der auf dem Patiententisch 17 anordbaren und/oder positionierbaren Zusatzeinheit umfasst ist. Die Absaugeinheit 510 weist ebenfalls zumindest eine Saugdüse 511, einen Lüfter 512 und/oder Ventilator und/oder eine Absaugpumpe, einen Absaugkanal 513, eine Ausblasöffnung 514 und einen Filter 515 auf, die ebenfalls von der Zusatzeinheit umfasst sind. Bezüglich einer Funktionsweise der Absaugeinheit 510 wird auf die Ausführungen zu den Fig. 3 bis 4 verwiesen.

Zur Reinigung der den Patientenaufnahmebereich 12 umgebenden Umhausung 14 wird zunächst die Zusatzeinheit der Reinigungseinheit 500 auf dem Patiententisch 17 positioniert. Für einen Reinigungsvorgang kann hierbei die Sprüheinheit 501 der Reinigungseinheit 500 während eines Einfahrens des Patiententischs 17 in den Patientenaufnahmebereich 12 aktiviert werden, so dass der Patientenaufnahmebereich 12, insbesondere die den Patientenaufnahmebereich 12 umgebende Umhausung 14, entlang einer gesamten Länge des Patientenaufnahmebereichs 12 mit dem Reinigungsmittel besprüht wird. Dabei kann gleichzeitig mit dem Einfahren des Patiententischs 17 in den Patientenaufnahmebereich 12 oder auch erst beim Ausfahren des Patiententischs 17 aus dem Patientenaufnahmebereich 12 die Absaugeinheit 510 aktiviert werden zu einem Absaugen der Reinigungsmittelaerosole aus dem Patientenaufnahmebereich 12. Während des Ausfahrens des Patiententischs 17 aus dem Patientenaufnahmebereichs 12 wird zudem die Trocknungseinheit 506 der Reinigungseinheit 500 aktiviert und derart der Patientenaufnahmebereich 12, insbesondere die den Patientenaufnahmebereich 12 umgebende Umhausung 14, getrocknet werden.

Die in den Fig. 1 bis 5 dargestellten Magnetresonanzvorrichtungen 10 können selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

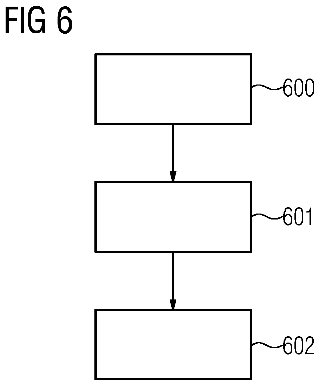

In Fig. 6 ist ein Verfahren zu einem Reinigen des Patientenaufnahmebereichs 12, insbesondere der den Patientenaufnahmebereich 12 umgebenden Umhausung 14, der Magnetresonanzvorrichtung 10 mittels einer der in den Fig. 2 bis 5 dargestellten Reinigungseinheit 200, 300, 400, 500 dargestellt. Sofern das Verfahren mit einer Reinigungseinheit 500, die zumindest teilweise als Zusatzeinheit ausgebildet ist, ausgeführt wird, wird vor Beginn des Verfahrens zu einem Reinigen der den Patientenaufnahmebereich 12 umgebenden Umhausung 14 von einem Benutzer, insbesondere einem medizinischen Bedienpersonal und/oder einem Reinigungspersonal, die Zusatzeinheit auf dem Patiententisch 17 der Patientenlagerungsvorrichtung 16 positioniert und/oder angeordnet. Dabei wird vom Benutzer die Zusatzeinheit derart auf dem Patiententisch 17 positioniert und/oder angeordnet, dass die Sprüheinheit 501, insbesondere die zumindest eine Düse 502 der Sprüheinheit 501, im Frontbereich 28 des Patiententischs 17 angeordnet ist.

Sofern die Reinigungseinheit 200, 300, 400 nach den Fig. 2 bis 4 ausgebildet ist, ist die Reinigungseinheit 200, 300, 400 zumindest teilweise bereits in dem Patiententisch 17 integriert, so dass keine weiteren Vorbereitungen zu einem Ausführen des Verfahrens zum Reinigen des Patientenaufnahmebereichs 12 erforderlich sind.

Zu Beginn wird von Benutzer das Verfahren zum Reinigen des Patientenaufnahmebereichs 12 gestartet. Dies kann beispielsweise mittels der Eingabeeinheit 26 der Benutzerschnittstelle 24 und/oder einer nicht näher dargestellten Bedieneinheit der Reinigungseinheit 200, 300, 400, 500 erfolgen. In einem ersten Verfahrensschritt 600 erfolgt ein Einfahren des Patiententischs 17 in den Patientenaufnahmebereich 12. Gleichzeitig mit dem Einfahren des Patiententischs 17 in den Patientenaufnahmebereichs 12 erfolgt in einem zweiten Verfahrensschritt 601 ein erster Reinigungsschritt eines Reinigungsvorgangs. Der erste Reinigungsschritt umfasst dabei ein Ausbringen und/oder eine Verteilen eines Reinigungsmittels mittels der Reinigungseinheit 200, 300, 400, 500. Insbesondere umfasst der ersten Reinigungsschritt ein Versprühen einer Reinigungsflüssigkeit mittels der Sprüheinheit 201, 301, 401, 501 der Reinigungseinheit 200, 300, 400, 500.

Ein dritter Verfahrensschritt 602 umfasst einen zweiten Reinigungsschritt des Reinigungsvorgangs, wobei der zweite Reinigungsschritt einen Trocknungsschritt und/oder einen Absaugschritt umfasst. Der Trocknungsschritt wird mittels der Trocknungseinheit 206, 306, 406, 506 der Reinigungseinheit 200, 300, 400, 500 ausgeführt. Der Absaugschritt wird mittels der Absaugeinheit 310, 410, 510 der Reinigungseinheit 300, 400, 500 ausgeführt.

Der dritte Verfahrensschritt 602, insbesondere der zweite Reinigungsschritt des Reinigungsvorgangs, kann dabei ebenfalls während des Einfahrens des Patiententischs 17 in den Patientenaufnahmebereichs 12 ausgeführt werden, insbesondere wenn der zweite Reinigungsschritt den Absaugschritt umfasst. Zudem kann der dritte Verfahrensschritt 602, insbesondere der zweite Reinigungsschritt des Reinigungsvorgangs, auch während eines Ausfahrens des Patiententischs 17 aus dem Patientenaufnahmebereich 12 ausgeführt werden, insbesondere wenn der zweiten Reinigungsschritt den Trocknungsschritt und/oder den Absaugschritt umfasst.

Bevorzugt läuft das Verfahren zu einem Reinigen des Patientenaufnahmebereichs 12, insbesondere der den Patientenaufnahmebereich 12 umgebenden Umhausung 14, nach einem einmaligen Starten durch einen Benutzer automatisch und/oder selbsttätig ab. Hierzu weist die Reinigungseinheit 100, 200, 300, 400, 500 eine Recheneinheit, die einen Ablauf des Verfahrens zu einem Reinigen der den Patientenaufnahmebereich 12 umgebenden Umhausung 14 steuert. Im vorliegenden ist die Recheneinheit der Reinigungseinheit 100, 200, 300, 400, 500 innerhalb der Systemsteuereinheit 23 der Magnetresonanzvorrichtung 10 integriert. Zudem kann die Recheneinheit der Reinigungseinheit auch separat zur Systemsteuereinheit 23 ausgebildet sein.

Zu einem Ausführen des Verfahrens zu einem Reinigen des Patientenaufnahmebereichs 12 weist die Recheneinheit der Reinigungseinheit 100, 200, 300, 400, 500 zumindest ein Rechenmodul und/oder einen Prozessor auf. So ist insbesondere die Recheneinheit dazu ausgebildet, computerlesbare Instruktionen auszuführen, um das erfindungsgemäße Verfahren zu einem Reinigen des Patientenaufnahmebereichs 12 auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen, um ein erfindungsgemäßes Verfahren zu einem Reinigen des Patientenaufnahmebereichs 12 auszuführen.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung mit einer Scannereinheit, einem von der Scannereinheit zumindest teilweise umgebenen Patientenaufnahmebereich, wobei die Scannereinheit eine den Patientenaufnahmebereich zumindest teilweise umgebende Umhausung umfasst, und einer Patientenlagerungsvorrichtung mit einem Patiententisch, wobei der Patiententisch zu einer horizontalen Bewegung innerhalb des Patientenaufnahmebereichs ausgebildet ist,
**gekennzeichnet durch** eine Reinigungseinheit zur Reinigung der den Patientenaufnahmebereich umgebenden Umhausung, wobei die Reinigungseinheit zumindest teilweise am Patiententisch angeordnet ist.

2. Magnetresonanzvorrichtung nach Anspruch 1,
**dadurch gekennzeichnet, dass** die Reinigungseinheit zumindest eine Einheit aufweist, die innerhalb des Patiententischs angeordnet ist und/oder als Zusatzeinheit auf dem Patiententisch anordbar und/oder positionierbar ist.

3. Magnetresonanzvorrichtung nach einem der vorhergehenden Ansprüche,
**dadurch gekennzeichnet, dass** die Reinigungseinheit eine Sprüheinheit aufweist, wobei die Sprüheinheit Düsen aufweist zu einem Verteilen eines Reinigungsmittels.

4. Magnetresonanzvorrichtung nach Anspruch 3,
**dadurch gekennzeichnet, dass** die Düsen in einem Frontbereich des Patiententischs angeordnet sind.

5. Magnetresonanzvorrichtung nach einem der Ansprüche 3 bis 4,
**dadurch gekennzeichnet, dass** die Sprüheinheit einen Vorratsbehälter aufweist, der an einer Basiseinheit der Patientenlagerungsvorrichtung angeordnet ist.

6. Magnetresonanzvorrichtung nach einem der vorhergehenden Ansprüche,
**dadurch gekennzeichnet, dass** die Reinigungseinheit eine Trocknungseinheit aufweist, wobei die Trocknungseinheit zu einem Einströmen einer Trocknungsluft in den Patientenaufnahmebereich ausgebildet ist.

7. Magnetresonanzvorrichtung nach Anspruch 6,
**dadurch gekennzeichnet, dass** die Trocknungseinheit zumindest teilweise am Patiententisch und/oder an der den Patientenaufnahmebereich umgebenden Umhausung angeordnet ist.

8. Magnetresonanzvorrichtung nach einem der vorhergehenden Ansprüche,
**dadurch gekennzeichnet, dass** die Reinigungseinheit eine Absaugeinheit aufweist.

9. Magnetresonanzvorrichtung nach Anspruch 8,
**dadurch gekennzeichnet, dass** die Absaugeinheit zumindest teilweise an der den Patientenaufnahmebereich umgebenden Umhausung und/oder am Patiententischs angeordnet ist.

10. Magnetresonanzvorrichtung nach einem der Ansprüche 8 bis 9,
**dadurch gekennzeichnet, dass** die Absaugeinheit zumindest einen Absaugkanal mit einem Filter umfasst.

11. Verfahren zu einem Reinigen eines Patientenaufnahmebereichs, insbesondere einer den Patientenaufnahmebereich umgebenden Umhausung, einer Magnetresonanzvorrichtung mittels einer Reinigungseinheit, wobei das Verfahren die folgenden Schritte umfasst:
- Einfahren eines Patiententischs in den Patientenaufnahmebereich, wobei an dem Patiententisch die Reinigungseinheit angeordnet ist,
- Gleichzeitig mit dem Einfahren des Patiententischs in den Patientenaufnahmebereich erfolgt ein Ausführen eines ersten Reinigungsschritts eines Reinigungsvorgangs, wobei der erste Reinigungsschritt ein Ausbringen eines Reinigungsmittels mittels der Reinigungseinheit umfasst, und
- Ausführen eines zweiten Reinigungsschritts, wobei der zweite Reinigungsschritt einen Trocknungsschritt und/oder einen Absaugschritt umfasst.

12. Verfahren nach Anspruch 11,
**dadurch gekennzeichnet, dass** der zweite Reinigungsschritt während des Einfahrens des Patiententischs in den Patientenaufnahmebereich und/oder während eines Ausfahrens des Patiententischs aus dem Patientenaufnahmebereich ausgeführt wird.
